# FASCICULE DE BREVET EUROPEEN

(11) **EP 4 092 511 B1**
(45) Date de publication et mention de la délivrance du brevet: **13.12.2023**
(21) Numéro de dépôt: 22174454.3
(22) Date de dépôt: 19.05.2022
(51) Int. Cl.: G06F 3/042, A61L 2/10, G06F 1/16, A61L 2/26

(54) **CADRE À ÉCLAIRAGE INFRAROUGE D' ÉCRAN TACTILE, DOTÉ DE DIODES UVC**
INFRAROTBELEUCHTETER TOUCHSCREEN-RAHMEN MIT UVC-DIODEN
FRAME WITH INFRARED LIGHTING FOR TOUCH SCREEN, PROVIDED WITH UVC DIODES

(30) Priorité: 22.05.2021 FR 2105367
(43) Date de publication de la demande: 23.11.2022
(73) Titulaire: Dreamtronic, 16000 Angouleme (FR)
(72) Inventeur: DEFAUX, Olivier, 24000 PERIGUEUX (FR)
(74) Mandataire: Aupetit, Muriel J. C.

(56) Documents cités:
- CN-U- 211 787 047
- KR-A- 20180 105 854
- KR-A- 20200 114 677
- US-A1- 2021 000 991
- US-A1- 2021 052 756

## Description

L'invention concerne un cadre à éclairage infrarouge d'écran tactile combinant un éclairage dans les ultraviolets-C, et un écran tactile intégrant un tel cadre.

La technologie infrarouge (IR) pour les écrans tactiles convient bien pour les écrans de grandes dimensions, notamment pour les écrans formant des bornes interactives dans les espaces publics, tels que des bornes de commande pour clients dans la restauration rapide, des consoles géantes tactiles ou tables tactiles de jeux, etc.

La technologie infrarouge permet de créer sur une surface, une matrice de faisceaux infrarouges s'étendant dans les deux directions X et Y en se croisant ; lorsque l'utilisateur touche l'écran, le point de contact est détecté car correspondant au point d'interruption de deux faisceaux. Aujourd'hui, un écran tactile employant la technologie IR comporte l'écran d'affichage à surface verrière sur laquelle peut être rapporté un substrat en polycarbonate (essentiellement pour la protection du verre de l'écran), et un cadre doté de diodes IR, qui est posé sur et à la périphérie du substrat externe de l'écran (sur le verre ou sur le polycarbonate lorsqu'il est présent), ainsi qu'une coque qui chausse le chant de l'ensemble précité et permet de cacher le cadre à IR.

Depuis l'apparition du coronavirus, il est devenu indispensable de proposer des solutions pour désinfecter les surfaces de ces écrans tactiles. Une solution est bien entendu de désinfecter manuellement ces surfaces, ce qui nécessite cependant, non seulement l'emploi de produits désinfectants et du papier jetable, mais aussi du personnel qui doit être mobilisé pour ces tâches sanitaires. Il existe également les solutions que l'on peut qualifier « d'auto-désinfectantes » pour lesquelles l'intervention humaine n'est pas nécessaire. Il est notamment connu d'utiliser des diodes à ultraviolets, en particulier dans le spectre des UVC, qui sont montées dans le cadre à IR des écrans tactiles. La demande de brevet WO2016051024A divulgue par exemple un écran tactile doté d'un cadre intégrant des diodes IR et des diodes UVC, les diodes UVC sont positionnées dans un plan supérieur à celui des diodes IR (c'est-à-dire dans un plan à l'opposé de l'écran et au plus près de l'utilisateur) et éclairent vers la surface tactile de l'écran en étant orientées de sorte à tenter de limiter un éclairage direct vers l'utilisateur, les UVC étant dangereux pour les yeux. Afin de s'assurer qu'aucun risque n'est pris pour l'utilisateur, les diodes UVC sont de préférence éteintes lors de l'utilisation de l'écran. Or, cette solution de n'allumer les diodes UVC pour désinfecter la surface tactile qu'en dehors de l'activité de l'écran, ne se révèle finalement pas optimale. En effet, un tel écran destiné au grand public peut dans certaines utilisations être sollicité quasiment en continu, et il ne sera alors pas suffisamment désinfecté. La demande US 2021/052756 A1 divulgue un appareil d'affichage, comportant un panneau d'affichage; un capteur tactile prévu sur le panneau d'affichage et configuré pour détecter une entrée tactile d'utilisateur; une source de lumière ultraviolette (UV) prévue sur une partie de bord du panneau d'affichage et configurée pour émettre une lumière UV au panneau d'affichage à partir de la partie de bord du panneau d'affichage; et un dispositif de commande connecté électriquement au panneau d'affichage, au capteur tactile, et à la source de lumière UV, le dispositif de commande étant configuré pour, sur la base d'une détermination que l'entrée tactile d'utilisateur n'est pas détectée, commander la source de lumière UV pour émettre la lumière UV vers le panneau d'affichage.

L'invention a donc pour but de proposer une solution de désinfection pour écran tactile à fonctionnement par IR, qui ne présente pas les inconvénients précités, en particulier qui permette d'assurer une désinfection efficace par UVC même en cours de fonctionnement de l'écran tactile sans risque pour les utilisateurs, qui soit simple de fabrication et puisse aisément être mise en oeuvre, y compris avec les écrans déjà existants.

L'invention est donc relative à un cadre à éclairage infrarouge d'écran tactile conforme à la revendication 1. Le cadre à éclairage infrarouge d'écran tactile présente sur son chant intérieur une ouverture longitudinale et comporte des diodes électroluminescentes émettant dans l'infrarouge, dites diodes IR, et des diodes électroluminescentes émettant dans l'ultraviolet-C, dites diodes UVC, les diodes IR et UVC étant agencées à l'intérieur du cadre et émettant leurs faisceaux en direction de l'ouverture (les diodes UVC étant agencées pour émettre des faisceaux dans un plan parallèle (ou sensiblement parallèle) au plan d'émission des faisceaux des diodes IR). De plus, les diodes IR sont agencées de manière décalée et vers l'ouverture par rapport aux diodes UVC, et le cadre comporte une surface de capotage qui est disposée entre les diodes IR et les diodes UVC, et qui chapeaute lesdites diodes UVC. Dans la suite de la description, les diodes IR s'entendent par les diodes IR émettrices et celles réceptrices ; les diodes IR émettrices sont disposées sur deux côtés perpendiculaires adjacents du cadre (pour fournir une matrice en coordonnées X, Y), et les diodes IR réceptrices sont disposées en regard sur les deux autres côtés opposés du cadre.

Ainsi, les diodes IR étant décalées vers l'avant de l'ouverture par rapport au diodes UVC, les diodes UVC sont par conséquent disposées vers le fond du cadre par rapport à l'ouverture du cadre (et destinées à être dans la partie inférieure du cadre par rapport aux diodes IR qui sont destinées à être en partie supérieure, et donc à l'opposé de l'utilisateur), et en étant chapeautées, il en résulte une protection réelle de l'utilisateur par rapport à l'émission des faisceaux UVC. De manière inattendue, cette configuration suffit à protéger l'utilisateur lorsque le cadre est opérationnel dans un écran tactile à IR, les diodes UVC étant en fonctionnement. Il a été constaté que le rayonnement UVC mesuré par un radiomètre à une hauteur de 3 cm au-dessus de l'écran est équivalent aux mesures de rayonnement du bruit de fond atmosphérique, ce qui ne présente ainsi aucun danger pour l'utilisateur.

Selon une caractéristique, les diodes IR et les diodes UVC sont respectivement disposées sur deux supports distincts (pour chacun des côtés du cadre). L'agencement des diodes sur deux supports distincts permet une fabrication simple et rapide du cadre, en utilisant notamment des diodes UVC du commerce déjà en place sur leur support. En outre, cela permet un remplacement aisé et moins coûteux en cas de dysfonctionnement de l'une des catégories de diodes et pas l'autre.

Selon une autre caractéristique, la surface de capotage est avantageusement formée par un support des diodes IR, de préférence le support des diodes IR étant une carte de circuit imprimé portant directement les diodes IR. Ainsi, la carte de circuit imprimé formant directement la surface de capotage, cela simplifie la conception du cadre.

Selon une caractéristique, le cadre comporte, du côté des diodes UVC et l'opposé de la surface de capotage, une surface (parallèle aux faisceaux des diodes UVC et au moins entre les diodes UVC et l'ouverture du cadre) qui est réfléchissante, notamment ladite surface réfléchissante étant métallique, en particulier en aluminium, de préférence constituée par le corps du cadre qui est métallique, en particulier en aluminium. D'une part, le cadre permet de chapeauter les diodes UVC qui sont placées en fond du cadre pour éviter un éclairage direct, et d'autre part, la surface réfléchissante en regard des diodes UVC (en-dessous des diodes UVC en position d'utilisation du cadre) favorise la réflexion des faisceaux en direction du volume intérieur délimité par le cadre, et donc en direction du centre de la surface tactile lorsque le cadre de l'invention est intégré à un écran tactile. De cette manière, tout en protégeant les yeux de l'utilisateur, le cadre est destiné à assurer une désinfection de la surface tactile de manière optimale non seulement sur les bords mais également jusqu'en son centre. La désinfection est efficace.

Selon une autre caractéristique, les diodes IR sont sur un support qui est perpendiculaire à un support portant les diodes UVC. Le support des diodes UVC est avantageusement disposé contre le fond du cadre (à l'opposé de l'ouverture, et contre la face interne du chant du cadre).

De préférence, les diodes IR et les diodes UVC sont sur des supports indépendants du cadre. De préférence, chacun des supports (directs)e des diodes IR et des diodes UVC sont amovibles par rapport au (corps du) cadre, de préférence le cadre comportant pour chaque côté doté de diodes IR et UVC, deux glissières dans lesquelles sont respectivement insérés les deux supports des diodes IR et des diodes UVC. En étant sur deux supports distincts qui sont amovibles, il est très facile de changer une série de diodes d'un support si certaines sont défectueuses tout en gardant les autres diodes sur l'autre support si celles-ci fonctionnent parfaitement. Avantageusement, les deux glissières sont conçues à partir d'une paroi en forme d'équerre dont le creux est en regard du fond (ou de l'âme du U du cadre) et destiné à accueillir le support direct des diodes UVC, et d'une autre paroi ou cloison perpendiculaire au fond (à l'âme du U) du cadre et parallèle à l'une des ailes de l'équerre pour ménager un espace destiné à l'accueil du support direct des diodes IR.

Avantageusement, le cadre comporte pour chaque côté doté de diodes UVC, un logement longitudinal accueillant le support des diodes UVC, le logement étant fermé dans un plan parallèle à l'ouverture longitudinale du cadre et comprenant une pluralité de fenêtres espacées et en regard desquelles sont disposées les diodes UVC. Ainsi, il n'est pas nécessaire d'agencer les diodes UVC de manière contigüe, on peut limiter le nombre de diodes, et via les fenêtres, canaliser chaque faisceau en direction de l'ouverture du cadre. En outre, il est obtenu un meilleur contrôle du rayonnement UV, procurant une sécurité encore meilleure d'utilisation. L'espacement des fenêtres est tel qu'en fonction de l'angle d'ouverture du faisceau d'une diode UVC, et la distance séparant les diodes UVC de l'ouverture du cadre, le recoupement des faisceaux se fasse au niveau de l'ouverture. De préférence, l'angle d'ouverture des diodes UVC est de 120°. A titre d'exemple les fenêtres sont espacées de l'ordre de 17 mm, et sont à une distance de l'ouverture longitudinale du cadre de 15 mm.

Selon une autre caractéristique, l'ouverture longitudinale peut être fermée par une bande (paroi longitudinale) rigide transparente, notamment montée amovible en étant glissée dans deux rainures opposées. On entend par « transparente », le fait de présenter une transmission lumineuse élevée pour laisser passer les faisceaux des diodes IR et UVC.

Il peut être préférable que les diodes UVC, ou une partie des diodes UVC, soient couplées à des lentilles. Une lentille peut être directement solidaire d'une diode UVC, ou bien être montée sur une fenêtre du logement du support des diodes UVC. En variante, la bande rigide transparente fermant l'ouverture du cadre peut posséder la fonction de lentille, uniquement en vis-à-vis des diodes UVC afin de ne pas perturber les faisceaux IR.

De préférence, le bord périphérique de l'ouverture longitudinale du cadre du côté des diodes IR est arrondi en présentant une concavité qui est tournée vers l'intérieur du cadre. Ce bord périphérique à arête arrondie est destiné à constituer le bord de l'écran tactile au plus près de l'utilisateur en position montée du cadre dans l'écran.

Selon encore une autre caractéristique, le cadre comporte plusieurs profilés, chacun des profilés constituant un côté du cadre, et des pièces de coins pour abouter les profilés, de préférence, les pièces de coin comportant des diodes UVC pour maximiser le rayonnement UVC. Les diodes UVC des pièces de coin sont disposées dans le même plan (à la même hauteur) que les diodes UVC des profilés.

Avantageusement, le cadre présente selon sa section transversale une forme en U, et comprend une âme opposée à l'ouverture et deux ailes opposées et parallèles perpendiculaires à l'âme. L'une des ailes par sa face interne constitue la surface réfléchissante associée aux diodes UVC.

De préférence, les pièces de coins sont montées amovibles.

Le cadre en étant fabriqué avec plusieurs profilés amovibles permet, en ne fabriquant que quelques modèles de profilés, de s'adapter à toutes tailles d'écrans déjà existants afin de changer leur cadre infrarouge avec celui de l'invention car possédant la fonction supplémentaire de désinfection de la surface tactile, et cela sans avoir à fabriquer une multitude de cadres par modèle d'écran. En outre, l'aspect amovible permet d'emballer le cadre de manière compact et de le transporter à destination lorsqu'il s'agit notamment de changer le cadre d'un écran existant ; il suffit sur place de monter les différents profilés pour former le cadre.

L'invention porte également sur un écran tactile à fonctionnement par IR comportant une dalle d'affichage (de surface externe généralement en verre), éventuellement recouverte d'un substrat transparent en matière plastique, et un cadre à éclairage infrarouge précité de l'invention, en particulier le cadre étant agencé de façon que les diodes UVC éclairent dans un plan au plus près de la dalle.

Dans la suite de la description, les qualificatifs « supérieur », « inférieur », « haut » et « bas » d'un élément et le terme « hauteur » sont utilisés dans le cadre d'une installation normale du cadre associé à un écran, l'écran étant disposé selon un plan horizontal. Les termes « avant » et « arrière » s'entendent par rapport à l'utilisation de l'écran et du cadre en position montée dans l'écran, la surface tactile étant vers l'avant de l'écran.

La présente invention est maintenant décrite à l'aide d'exemples uniquement illustratifs et nullement limitatifs de la portée de l'invention, et à partir des illustrations jointes, dans lesquelles :
- [Fig. 1] ou figure 1 représente une vue en perspective d'un cadre à éclairage infrarouge selon l'invention pourvu également d'une fonction désinfectante via des diodes UVC.
- [Fig. 2] ou figure 2 représente une vue en coupe partielle schématique d'un écran tactile incorporant le cadre à éclairage infrarouge de la figure 1.
- [Fig. 3] ou figure 3 est une vue partielle en perspective du cadre à éclairage infrarouge des figures 1 et 2.
- [Fig. 4] ou figure 4 est une vue en perspective éclatée de deux profilés adjacents du cadre de la figure 1 reliés mécaniquement par une pièce de coin.
- [Fig. 5] ou figure 5 correspond à la figure 3 sans les diodes IR et UVC.
- [Fig. 6] ou figure 6 montre une autre vue légèrement en perspective d'un profilé sans les diodes et d'une pièce de coin assemblée au profilé.

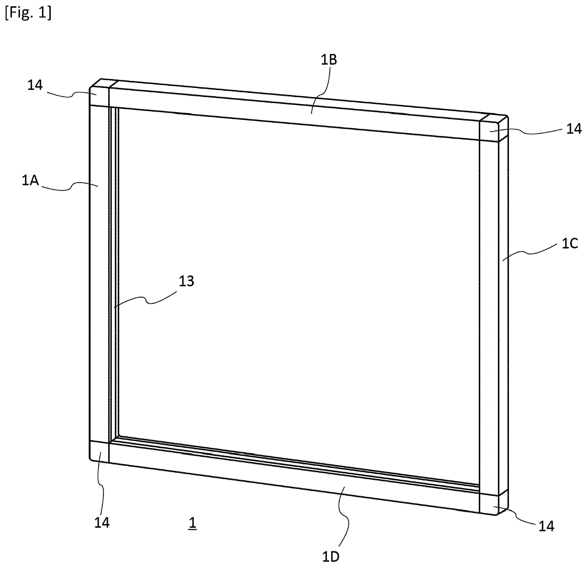

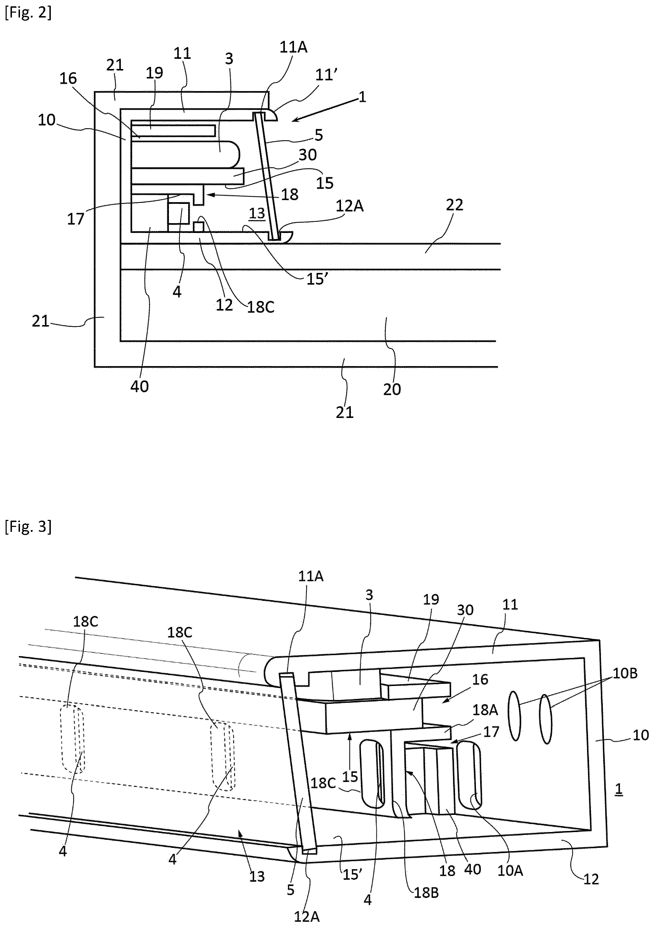

Le cadre d'écran tactile à éclairage infrarouge 1 de l'invention illustré sur la figure 1 est destiné à être intégré à un écran tactile 2 tel que montré sur la figure 2, l'écran tactile 2 fonctionnant ainsi avec la technologie infrarouge (IR). L'écran tactile 2 est destiné à être auto-désinfecté au niveau de sa surface d'affichage (qui est celle touchée par l'utilisateur) grâce à des diodes UVC intégrées au cadre à éclairage infrarouge 1.

L'écran tactile 2 comporte une dalle d'affichage 20 dont la face avant forme la surface dite tactile (car touchée par l'utilisateur), le cadre à éclairage infrarouge 1 qui est agencé à la périphérie de la dalle 20, et une coque 21 qui chausse le cadre à éclairage infrarouge 1 et le chant périphérique de la dalle 20 afin d'assurer l'assemblage de la dalle et du cadre, de protéger ledit cadre et procurer l'esthétisme voulu à l'écran tactile. Selon le modèle d'écran tactile, la coque 21 peut également englober toute la surface arrière de la dalle 20. Le module électronique de fonctionnement de l'écran tactile et la connectique sont disposés à l'arrière de l'écran. La dalle 20 peut comporter sur sa face avant, en particulier pour les écrans de grandes dimensions, un substrat de protection 22 transparent en matière plastique tel qu'en polycarbonate.

Le cadre à éclairage infrarouge 1 est généralement rectangulaire et comporte le long de ses quatre côtés des diodes IR 3 émettant et recevant dans l'infrarouge qui, lorsqu'elles éclairent, forment un maillage avec une multitude de points de coordonnées X, Y. Lorsqu'un utilisateur touche la dalle 20 de l'écran, le point de contact est repéré par les coordonnées du point d'interruption des faisceaux infrarouges. L'éclairage IR s'étend dans un plan au plus près de l'utilisateur. On entend par « dalle 20 de l'écran », la surface directe de la dalle 20 ou le substrat 22 en matière plastique lorsqu'il est présent.

Le cadre à éclairage infrarouge 1 est également à fonction désinfectante pour supprimer bactéries, virus et spores sur la surface avant de la dalle 20, notamment supprimer toute trace du coronavirus humain. A cet effet, le cadre à éclairage infrarouge 1 comporte des diodes UVC 4. Les diodes UVC 4 éclairent en étant agencées, en position montée du cadre à éclairage infrarouge 1 dans l'écran 2, dans un plan en-dessous des diodes IR 4 et au plus près de la surface d'affichage à désinfecter (figure 2), et en direction de l'intérieur du cadre et donc du centre de l'écran.

Comme montré sur les figures 2, 3 et 5, le cadre à éclairage infrarouge 1 présente avantageusement une section transversale de forme en U, en comprenant une âme 10 formant le chant extérieur du cadre et deux ailes 11 et 12 parallèles et en regard qui sont perpendiculaires à l'âme 10. Les ailes 11 et 12 sont destinées à être dans des plans parallèles à la surface tactile de la dalle 20. Les diodes IR 3 et les diodes UVC 4 sont logées à l'intérieur du U. Le cadre à éclairage infrarouge 1 comporte une ouverture longitudinale 13 sur son chant longitudinal à l'opposé de l'âme 10. L'ouverture 13 est tournée vers l'intérieur du volume intérieur délimité par ledit cadre, c'est-à-dire destiné à être tournée vers la dalle 20.

De préférence, au moins l'arête 11' du bord de l'aile 11 du cadre 1 (de l'ensemble de la périphérie de cadre, et du côté des diodes IR et du côté utilisateur en position d'utilisation du cadre) est arrondie avec une concavité tournée vers l'intérieur du cadre.

Les diodes IR 3 présentent leur faisceau qui s'étend en direction de l'ouverture 13 et dans un plan parallèle aux ailes 11 et 12 du cadre afin qu'en position d'utilisation du cadre, les faisceaux desdites diodes IR 3 soient parallèles à la surface tactile de la dalle 20. Les diodes IR 3 sont disposées du côté de l'aile 11 destinée à être en partie supérieure du cadre au plus près de l'utilisateur.

Les diodes UVC 4 présentent également leur faisceau qui s'étend en direction de l'ouverture 13 et dans un plan parallèle aux ailes 11 et 12 du cadre afin qu'en position d'utilisation du cadre, les faisceaux desdites diodes UVC soient parallèles à la surface tactile de la dalle 20 et au plus près de la dalle. Les diodes IR 3 et les diodes UVC 4 sont agencées de sorte que leurs faisceaux soient dans des plans espacés et parallèles. Les diodes UVC 4 sont disposées du côté de l'aile 12 qui est destinée à être en partie inférieure du cadre au plus proche de la dalle 20. En position montée du cadre à éclairage infrarouge 1 dans l'écran tactile 2, les diodes IR 3 éclairent dans un plan au plus près de l'aile 11 proche de l'utilisateur et à l'opposé de la dalle 20, tandis que les diodes UVC 4 éclairent dans un plan en-dessous des diodes IR 3 au plus près de la dalle, et en arrière du plan vertical des diodes IR 3.

Le cadre à éclairage infrarouge 1 peut être monolithique, c'est-à-dire fait d'un profilé unitaire délimitant l'ensemble de la périphérie rectangulaire, l'ensemble des composants ayant par exemple été encapsulés. De préférence, le cadre à éclairage infrarouge 1 n'est pas monobloc; il comporte (figure 1) plusieurs profilés aboutés, quatre profilés 1A, 1B, 1C, 1D correspondant aux quatre côtés respectifs du cadre. Les profilés 1A à 1D sont de préférence montés amovibles les uns par rapport aux autres et raccordés par des pièces de jonction 14. Les pièces de jonction 14 montrées ici sont des pièces de coin. Les pièces de jonction 14 pourraient également former en supplément, des pièces linéaires pour relier deux profilés sur un même côté. L'amovibilité des profilés et l'utilisation de pièces de jonction 14 permet de minimiser le volume d'emballage et de transport du cadre, de remplacer facilement l'un des profilés si ultérieurement seule une partie des diodes sur un côté du cadre est endommagée, et si besoin de rendre modulable les dimensions du cadre à éclairage infrarouge 1.

Les diodes IR 3 sont disposées sur un premier support 30, et les diodes UVC 4 sont disposées sur un second support 40 distinct. De préférence, les supports 30 des diodes IR et 40 des diodes UVC ont une forme générale parallélépipédique plate, s'étendant selon la longueur de chacun des côtés du cadre.

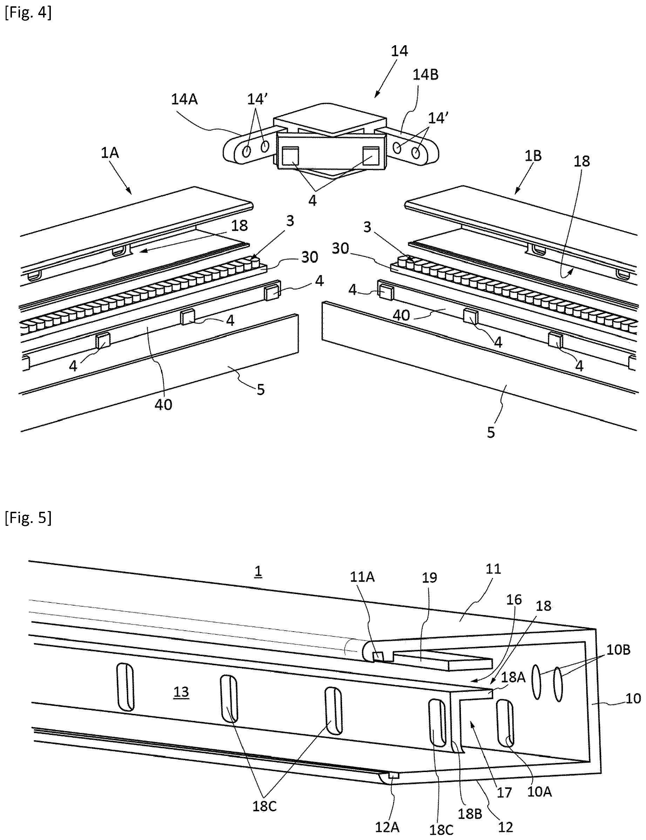

Le support 30 des diodes IR est de préférence indépendant du cadre et amovible. Le support 30 est de préférence directement une carte à circuit imprimé sur laquelle sont soudées et connectées électriquement les diodes IR 3. Comme montré sur la figure 4, les diodes IR 3 sont serrées les unes contre les autres (quasiment de manière jointive). Le support 30 des diodes IR est monté de façon à être parallèle aux ailes 11 et 12 du cadre (perpendiculaire à l'ouverture 13). Le support 30 des diodes IR est en regard de l'aile (supérieure) 11 destinée à être au plus près de l'utilisateur (de l'avant du cadre de l'écran).

Le support 40 des diodes UVC est de préférence un ensemble monobloc, indépendant du cadre et amovible. Le support 40 est par exemple une bande sur laquelle sont fixées les diodes UVC 4. Une telle bande se trouve dans le commerce. Les diodes UVC 4 sont espacées régulièrement sans besoin d'être rapprochées comme les diodes IR 3. Le nombre de diodes UVC 4 et leur écartement sont notamment fonction de l'angle d'ouverture de leur faisceau. De manière préférée, les diodes UVC 4 présentent un angle d'ouverture de 120°. Elles sont par exemple distantes les unes des autres de l'ordre de 33 mm.

Les diodes UVC 4 sont agencées du côté de l'aile 12 (inférieure du cadre) destinée à être au plus près de la surface tactile de la dalle 20. Les diodes UVC 4 sont agencées dans le fond de l'âme 10 du U du cadre, ce qui évite un éclairage direct vers l'utilisateur. Le support 40 des diodes UVC est avantageusement disposé de manière parallèle au fond 10 et de manière perpendiculaire au support 30 des diodes IR. Les diodes IR 3 sont décalées par rapport au diodes UVC 4 et vers l'ouverture 13. Avantageusement, le cadre 1 comporte une surface de capotage 15 qui s'étend au-dessus des diodes UVC 4 et en direction de l'ouverture 13. Bien que les diodes UVC 4 soient à l'opposé de la tranche intérieure du cadre, c'est-à-dire à l'opposé de l'utilisateur pour éviter un éclairage direct, cette surface de capotage 15 permet réellement de garantir que le rayonnement UVC ne sera pas nocif à l'utilisateur tout en assurant une désinfection de la surface tactile de la dalle en canalisant, en guidant les faisceaux vers le centre du cadre 1 et donc vers le centre de la dalle 20. De préférence, cette surface de capotage 15 est directement formée par le support 30 des diodes IR 3, lui-même directement formé de préférence par le circuit imprimé des diodes IR 3, ce qui simplifie la conception.

Le cadre à éclairage infrarouge 1, en particulier chacun des profilés 1A à 1D, comporte en aval des diodes UVC 4 (en direction de l'ouverture 13) et, en position montée du cadre dans l'écran, dans un plan en-dessous des diodes UVC 4 et à l'opposé de la surface de capotage 15, une surface réfléchissante 15' qui s'étend dans un plan parallèle aux faisceaux des diodes UVC 4. Cette surface réfléchissante 15' couvre en aval ou en avant des diodes UVC 4, toute la surface de l'aile 12 du profilé qui est opposée à la surface de capotage 15, c'est-à-dire à l'opposé du support 30 des diodes IR 3. De préférence, la surface réfléchissante 15' est directement constituée par la surface du profilé qui est métallique, en particulier en aluminium. Cette surface réfléchissante 15' (pourtant en-dessous des diodes UVC et dans un plan extrêmement proche du plan de la dalle) combinée à la surface de capotage 15 canalise le rayonnement UVC et participe encore mieux à sa diffusion en direction de la surface tactile de la dalle 20 et vers le milieu de la dalle, tout en évitant un éclairage direct de l'utilisateur. Les diodes UVC 4 peuvent fonctionner sans risque en continu et durant le fonctionnement de l'écran tactile.

La surface réfléchissante 15' participe grandement à faire parvenir les faisceaux UVC jusqu'au centre de la dalle tactile. En adaptant le nombre de diodes UVC 4 (selon également de leur angle d'ouverture) en fonction de la surface à désinfecter, le cadre à éclairage infrarouge 1 de l'invention peut fournir une puissance notamment de 30 W/cm², ce qui permet une désinfection rapide d'un écran tactile de 22 pouces, en 25 secondes en ses bords et en 40 secondes en son centre (données mesurées pour une désinfection du COVID 19). Le cadre à éclairage infrarouge 1 de l'invention permet d'utiliser l'écran, alors même que les diodes UVC éclairent, sans risque majeur pour l'utilisateur. Bien entendu, les diodes UVC 4 peuvent être couplées à un programmateur et à des moyens de détection de fonctionnement de l'écran pour n'allumer les diodes UVC qu'en dehors de l'utilisation de l'écran tactile.

Le cadre à éclairage infrarouge 1 et les profilés 1A à 1D le constituant sont conçus pour assurer un montage et un démontage aisés et rapides des diodes IR et UVC respectivement sur les supports 30 et 40 facilitant toute opération de maintenance.

De préférence, comme illustré sur la figure 3 et la figure 5, le cadre à éclairage infrarouge 1 comportent une première glissière 16 pour accueillir le support 30 des diodes IR 3, et une seconde glissière 17 pour loger le support 40 des diodes UVC 4. Chaque profilé 1A à 1D est fabriqué de la même manière avec deux glissières 16 et 17. Les deux glissières sont conçues pour loger les deux supports amovibles respectifs 30 et 40 sans moyens de fixation rapportés. Les dimensions des glissières 16 et 17 sont ainsi adaptées pour procurer un maintien des deux supports 30 et 40 dans chaque profilé alors que le profilé peut être manipulé sans risque que les deux supports ne glissent, ne tombent. Les supports directs 30 et 40 des diodes IR et UVC sont insérés légèrement en force dans les glissières respectives 16 et 17.

Dans l'exemple préféré illustré sur les figures, la seconde glissière 17 destinée à accueillir les diodes UVC 4 est procurée par une forme longitudinale en équerre 18 agencée contre l'âme 10 du profilé, l'intérieur de l'équerre étant en regard de l'âme 10 du profilé ce qui ménage un logement creux d'accueil formant ladite glissière 17 du support 40 des diodes UVC. La forme en équerre 18 comporte deux ailes 18A et 18B perpendiculaires l'une à l'autre, la première aile 18A s'appuyant perpendiculairement contre l'âme 10 du profilé à distance de l'aile inférieure 12, tandis que la seconde aile 18B perpendiculaire à la première aile 18A rejoint ladite aile inférieure 12 du profilé (figure 3). Une fois le support 40 des diodes UVC 4 logé dans la seconde glissière 17, ledit support 40 est parallèle à la seconde aile 18B de la forme en équerre (figure 3), et est donc parallèle à l'ouverture 13 du profilé. La seconde aile 18B de la forme en équerre du profilé comporte une pluralité de fenêtres 18C qui sont régulièrement espacées pour correspondre à l'écartement des diodes UVC 4. En position montée du support 40 des diodes UVC, les fenêtres 18C sont en vis-à-vis des diodes UVC 4. Les fenêtres 18C permettent de focaliser les faisceaux en sortie des diodes UVC 4.

Quant à la première glissière 16 accueillant le support 30 des diodes IR, elle est formée par la première aile 18A de la forme en équerre, celle orthogonale à l'âme 10 du profilé, et d'une cloison 19 s'étendant depuis l'âme 10 en direction de l'ouverture 13 et parallèlement à ladite première aile 18A et en regard de l'aile supérieure 11 du cadre. La première aile 18A et la cloison 19 sont espacées l'une de l'autre pour correspondre à l'épaisseur du support 30 des diodes IR 3, qui s'insère par glissement longitudinal ou par emboîtement dans le sens de l'épaisseur dudit support.

En outre, l'âme 10 comporte au moins à l'une des extrémités de chaque profilé, un trou 10A pour le passage des câbles de connexion électrique des deux supports 3 et 4 des diodes IR et UVC.

De préférence, les ou une partie des diodes UVC 4 sont couplées à des lentilles.

Une plaque 5 est ici disposée dans le plan de l'ouverture 13. En particulier, la plaque 5 ferme de manière transparente l'ouverture 13. Elle permet de protéger les diodes IR 3 et les diodes UVC 4 de la poussière et d'éviter à l'utilisateur d'insérer ses doigts. La plaque 5 est de préférence amovible et est fixée par glissement dans deux rainures opposées 11A et 12A qui sont établies dans respectivement chacune des ailes 11 et 12 du profilé et au bord de l'ouverture 13.

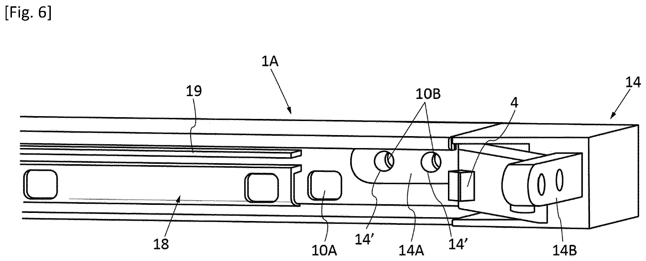

Par ailleurs, l'assemblage des profilés 1A à 1D se fait via les pièces de coin 14. Chaque pièce de coin 14 (figure 4 et figure 6) comporte deux pattes de fixation 14A et 14B en équerre. Chaque extrémité de profilé comporte, en particulier dans l'âme 10, des formes adaptées 10B pour coopérer de manière amovible avec les pattes de fixation 14A et 14B. Chacune des pattes de fixation 14A, 14B comportent des orifices 14' qui coopèrent avec les formes adaptées 10B, ici des orifices, des extrémités de profilés, pour être ensuite boulonnées. En variante, les pièces de coins 14 et les profilés 1A à 1D peuvent être assemblés par clippage ; chacune des pattes de fixation 14A, 14B et les extrémités de profilés comportent des formes par coopération mutuelle.

De préférence, chaque pièce de coin 14 présente des diodes UVC 4 qui sont agencées dans le même plan que celui des autres diodes UVC 4 des profilés et selon la diagonale reliant les deux pattes de fixation 14A et 14B (et donc selon la diagonale reliant les supports 40 de diodes UVC de deux profilés adjacents).

## Revendications

1. Cadre (1) à éclairage infrarouge d'écran tactile présentant sur son chant intérieur une ouverture (13) longitudinale, et des diodes électroluminescentes émettant dans l'infrarouge, dites diodes IR (3), et des diodes électroluminescentes émettant dans l'ultraviolet-C, dites diodes UVC (4), les diodes IR et les diodes UVC étant agencées à l'intérieur du cadre et émettant leurs faisceaux en direction de l'ouverture, **caractérisé en ce que** les diodes IR (3) sont agencées de manière décalée et vers l'ouverture (13) par rapport aux diodes UVC (4), le cadre (1) comporte une surface de capotage (15) qui est disposée entre les diodes IR (3) et les diodes UVC (4) et qui chapeaute lesdites diodes UVC (4), et **en ce qu'**il comporte au moins pour un côté doté des diodes UVC (4), un logement (17) longitudinal accueillant les diodes UVC (4), qui est fermé dans un plan parallèle à l'ouverture (13) et comprend une pluralité de fenêtres (18C) espacées et en regard desquelles sont disposées les diodes UVC (4).

2. Cadre selon la revendication 1, **caractérisé en ce que** les diodes IR (3) et les diodes UVC (4) sont respectivement disposées sur deux supports distincts (30, 40).

3. Cadre selon la revendication 1 ou 2, **caractérisé en ce que** la surface de capotage (15) est formée par un support (30) des diodes IR (3), de préférence le support (30) des diodes IR (3) étant une carte de circuit imprimé portant directement les diodes IR (3).

4. Cadre selon l'une quelconque des revendications précédentes, **caractérisé en ce qu'**il comporte, du côté des diodes UVC (4) et l'opposé de la surface de capotage (15), une surface (15') qui est réfléchissante, notamment ladite surface réfléchissante étant métallique, en particulier en aluminium, de préférence constituée par le corps du cadre qui est métallique, en particulier en aluminium.

5. Cadre selon l'une quelconque des revendications précédentes, **caractérisé en ce que** les diodes IR (3) sont sur un support (30) qui est perpendiculaire à un support (40) portant les diodes UVC (4).

6. Cadre selon l'une quelconque des revendications précédentes, **caractérisé en ce que** les diodes IR (3) et les diodes UVC (4) sont sur des supports (30, 40) indépendants du cadre et sont amovibles par rapport au cadre, de préférence le cadre (1) comportant pour chaque côté doté de diodes IR et UVC, deux glissières (16, 17) dans lesquelles sont respectivement insérés les deux supports (30, 40) des diodes IR et des diodes UVC.

7. Cadre selon l'une quelconque des revendications précédentes, **caractérisé en ce que** l'angle d'ouverture des diodes UVC est de 120°.

8. Cadre selon l'une quelconque des revendications précédentes, **caractérisé en ce que** l'ouverture (13) longitudinale est fermée par une bande rigide transparente (5), notamment montée amovible en étant glissée dans deux rainures opposées (11A, 12A).

9. Cadre selon l'une quelconque des revendications précédentes, **caractérisé en ce qu'**il comporte plusieurs profilés (1A, 1B, 1C, 1D), chacun des profilés constituant un côté du cadre, et des pièces de coins (14) pour abouter les profilés, de préférence, les pièces de coin (14) comportant des diodes UVC (4).

10. Cadre selon la revendication précédente, **caractérisé en ce que** les profilés (1A, 1B, 1C, 1D) et les pièces de coin (14) sont amovibles entre eux.

11. Cadre selon l'une quelconque des revendications précédentes, **caractérisé en ce que** les diodes UVC (4), ou une partie des diodes UVC, sont couplées à des lentilles, une lentille étant directement solidaire d'une diode UVC, ou bien étant montée sur une fenêtre (18C).

12. Ecran tactile (2) à fonctionnement par IR comportant une dalle d'affichage (20), éventuellement recouverte d'un substrat transparent en matière plastique (22), et un cadre (1) à éclairage infrarouge selon l'une quelconque des revendications précédentes, en particulier le cadre (1) étant agencé de façon que les diodes UVC (4) soient dans un plan à l'opposé de la dalle (20), dans la partie inférieure du cadre par rapport aux diodes IR (3) qui sont en partie supérieure.

13. Ecran tactile (2) à fonctionnement par IR comportant une dalle d'affichage (20), éventuellement recouverte d'un substrat transparent en matière plastique (22), et un cadre (1) à éclairage infrarouge selon l'une quelconque des revendications 1 à 11, **caractérisé en ce que** l'éclairage des diodes UVC (4) est configuré pour désinfecter la surface tactile de la dalle (20).

14. Ecran tactile (2) selon la revendication précédente, **caractérisé en ce qu'**il est configuré pour être utilisé alors même que les diodes UVC (14) éclairent.

## Patentansprüche

1. Rahmen (1) mit Infrarotbeleuchtung eines Touchscreens, der auf seiner Innenkante eine Längsöffnung (13) aufweist und Licht emittierende Dioden, die im Infrarotbereich emittieren, IR-Dioden (3) genannt, und Licht emittierende Dioden, die im Ultraviolett-C-Bereich emittieren, UVC-Dioden (4) genannt, wobei die IR-Dioden und die UVC-Dioden in dem Inneren des Rahmens eingerichtet sind und ihre Bündel in Richtung der Öffnung emittieren, **dadurch gekennzeichnet, dass** die IR-Dioden (3) versetzt und zur Öffnung (13) hin in Bezug auf die UVC-Dioden (4) eingerichtet sind, der Rahmen (1) eine Verkleidungsoberfläche (15) umfasst, die zwischen den IR-Dioden (3) und den UVC-Dioden (4) eingerichtet ist und die UVC-Dioden (4) abdeckt, und dass er mindestens für eine Seite, die mit UVC-Dioden (4) versehen ist, eine Längsaufnahme (17) umfasst, die die UVC-Dioden (4) aufnimmt, die in einer Ebene parallel zu der Öffnung (13) geschlossen ist und eine Vielzahl beabstandeter Fenster (18C) umfasst und welchen gegenüber die UVC-Dioden (4) angeordnet sind.

2. Rahmen nach Anspruch 1, **dadurch gekennzeichnet, dass** die IR-Dioden (3) und die UVC-Dioden (4) jeweils auf zwei getrennten Trägern (30, 40) angeordnet sind.

3. Rahmen nach Anspruch 1 oder 2, **dadurch gekennzeichnet, dass** die Verkleidungsoberfläche (15) durch einen Träger (30) der IR-Dioden (3) gebildet ist, wobei der Träger (30) der IR-Dioden (3) bevorzugt eine Leiterplatte ist, die die IR-Dioden (3) direkt trägt.

4. Rahmen nach einem der vorstehenden Ansprüche, **dadurch gekennzeichnet, dass** er auf der Seite der UVC-Dioden (4) und der Verkleidungsoberfläche (15) entgegengesetzt eine Oberfläche (15') umfasst, die reflektierend ist, wobei die reflektierende Oberfläche insbesondere metallisch ist, insbesondere aus Aluminium besteht, bevorzugt von dem Körper des Rahmens, der metallisch ist, gebildet ist, insbesondere aus Aluminium.

5. Rahmen nach einem der vorstehenden Ansprüche, **dadurch gekennzeichnet, dass** die IR-Dioden (3) auf einem Träger (30) liegen, der zu einem Träger (40), der die UVC-Dioden (4) trägt, senkrecht ist.

6. Rahmen nach einem der vorstehenden Ansprüche, **dadurch gekennzeichnet, dass** die IR-Dioden (3) und die UVC-Dioden (4) auf Trägern (30, 40) liegen, die von dem Rahmen unabhängig und in Bezug auf den Rahmen abnehmbar sind, wobei der Rahmen (1) bevorzugt für jede Seite, die mit IR-Dioden und UVC-Dioden versehen ist, zwei Gleitschienen (16, 17) umfasst, in welchen jeweils die zwei Träger (30, 40) der IR-Dioden und der UVC-Dioden eingefügt sind.

7. Rahmen nach einem der vorstehenden Ansprüche, **dadurch gekennzeichnet, dass** der Öffnungswinkel der UVC-Dioden 120° beträgt.

8. Rahmen nach einem der vorstehenden Ansprüche, **dadurch gekennzeichnet, dass** die Längsöffnung (13) durch ein starres durchsichtiges Band (5) verschlossen ist, das insbesondere abnehmbar montiert ist, indem es in zwei einander entgegengesetzte Nuten (11A, 12A) geschoben ist.

9. Rahmen nach einem der vorstehenden Ansprüche, **dadurch gekennzeichnet, dass** er mehrere Profile (1A, 1B, 1C, 1D) umfasst, wobei jedes der Profile eine Seite des Rahmens bildet, und Eckstücke (14), um die Profile aneinander zu fügen, wobei die Eckstücke (14) bevorzugt UVC-Dioden (4) umfassen.

10. Rahmen nach dem vorstehenden Anspruch, **dadurch gekennzeichnet, dass** die Profile (1A, 1B, 1C, 1D) und die Eckstücke (14) voneinander abnehmbar sind.

11. Rahmen nach einem der vorstehenden Ansprüche, **dadurch gekennzeichnet, dass** die UVC-Dioden (4) oder ein Teil der UVC mit Linsen gekoppelt sind, wobei eine Linse direkt fest mit einer UVC-Diode verbunden ist, oder aber auf ein Fenster (18C) montiert ist.

12. IR-betriebener Touchscreen (2), der eine Anzeigeplatte (20) umfasst, die eventuell mit einem durchsichtigen Substrat aus Kunststoff (22) abgedeckt ist, und einen Rahmen (1) mit Infrarotbeleuchtung nach einem der vorstehenden Ansprüche, wobei der Rahmen (1) insbesondere derart eingerichtet ist, dass die UVC-Dioden (4) in einer Ebene, die der Platte (20) entgegengesetzt ist, in dem unteren Teil des Rahmens in Bezug auf die IR-Dioden (3), die sich im oberen Bereich befinden, liegen.

13. IR-betriebener Touchscreen (2), der eine Anzeigeplatte (20) umfasst, die eventuell mit einem durchsichtigen Substrat aus Kunststoff (22) abgedeckt ist, und einen Rahmen (1) mit Infrarotbeleuchtung nach einem der Ansprüche 1 bis 11, **dadurch gekennzeichnet, dass** die Beleuchtung der UVC (4) dazu konfiguriert ist, die Berührungsoberfläche der Platte (20) zu desinfizieren.

14. Touchscreen (2) nach dem vorstehenden Anspruch, **dadurch gekennzeichnet, dass** er dazu konfiguriert ist, verwendet zu werden, auch wenn die UVC-Dioden (14) beleuchten.

## Claims

1. An IR-lighting frame (1) for a touchscreen, which has, along its inner edge, a longitudinal opening (13) and electroluminescent diodes that emit IR-rays, called IR diodes (3), and electroluminescent diodes that emit UVC-rays, called UVC diodes (4), wherein the IR diodes and the UVC diodes are arranged inside the frame and emit rays toward the opening, **characterized in that** the IR diodes (3) are arranged shifted toward the opening (13) relatively to the UVC diodes (4), the frame (1) has a cowling surface (15) between the IR diodes (3) and the UVC diodes (4) that covers said UVC diodes (4), and **in that** it comprises, at least on a side with UVC diodes (4), a longitudinal recess (17) that receives the UVC diodes (4) and that is closed in a plane parallel to the opening (13) and that comprises a plurality of spaced windows (18C) with the UVC diodes (4) in front of them.

2. A frame according to claim 1, **characterized in that** the IR diodes (3) and the UVC diodes (4) are respectively located on two distinct mounts (30, 40).

3. A frame according to claim 1 or 2, **characterized in that** the cowling surface (15) is made of a mount (30) for the IR diodes (3), wherein the mount (30) for the IR diodes (3) is preferably a printed circuit board with the IR diodes (3) directly mounted on it.

4. A frame according to any one of previous claims, **characterized in that** it comprises, on the side of the UVC diodes (4) and opposite to the cowling surface (15), a reflecting surface (15'), in particular wherein said reflecting surface is metallic, in particular made of aluminum, preferably made of the body of the frame that is metallic, in particular made of aluminum.

5. A frame according to any one of previous claims, **characterized in that** the IR diodes (3) are located on a mount (30) that is perpendicular to a mount (40) with the UVC diodes (4) on it.

6. A frame according to any one of previous claims, **characterized in that** the IR diodes (3) and the UVC diodes (4) are on mounts (30, 40) that are independent from the frame and are removable from the frame, wherein preferably the frame (1) comprises, for each side with IR and UVC diodes on it, two slides (16, 17) where the two mounts (30, 40) for the IR diodes and UVC diodes are inserted.

7. A frame according to any one of the previous claims, **characterized in that** the opening angle of the UVC diodes is 120°.

8. A frame according to any one of previous claims, **characterized in that** the longitudinal opening (13) is closed by a transparent rigid band (5), in particular removably mounted by sliding it into two opposite grooves (11A, 12A).

9. A frame according to any one of previous claims, **characterized in that** it comprises several profiles (1A, 1B, 1C, 1D), wherein each profile constitutes a side of the frame, and corner parts (14) to join the profiles together, preferably corner parts (14) comprising UVC diodes (4).

10. A frame according to the previous claim, **characterized in that** the profiles (1A, 1B, 1C, 1D) and the corner parts (14) are mutually removable.

11. A frame according to any one of previous claims, **characterized in that** the UVC diodes (4) or a part of the UVC diodes are coupled to lenses, wherein a lens is directly sticked together with an UVC diode or is mounted on a window (18C).

12. A touchscreen (2) working with IR that comprises a display faceplate (20), possibly covered with a transparent plastic substrate (22), and a frame (1) with IR lighting according to any one of the previous claims, in particular wherein the frame (1) is designed so that the UVC diodes (4) are in a plane opposite to the faceplate (20) in a lower part of the frame relatively to the IR diodes (3) that are in an upper part.

13. A touchscreen (2) working with IR that comprises a display faceplate (20), possibly covered with a transparent plastic substrate (22), and a frame (1) with IR lighting according to any one of the claims 1 to 11, **characterized in that** the lighting of the UVC diodes (4) is configured to disinfect the touch surface of the faceplate (20).

14. A touchscreen (2) according to the previous surface, **characterized in that** it is configured for use while the UVC diodes (14) are lighting.
